(19) Europäisches Patentamt
European Patent Office
Office européen des brevets

(11) Veröffentlichungsnummer : **0 103 896**
**B1**

(12) **EUROPÄISCHE PATENTSCHRIFT**

(45) Veröffentlichungstag der Patentschrift : **17.07.85**

(21) Anmeldenummer : **83109399.2**

(22) Anmeldetag : **21.09.83**

(51) Int. Cl.⁴ : **C 07 C 47/228**, C 07 C 45/68, C 07 C 87/29, C 07 D295/02, C 07 D263/04, C 07 C119/10

(54) **Verfahren zur Herstellung eines araliphatischen Aldehyds und Mittel zu dessen Herstellung.**

(30) Priorität : **21.09.82 CH 5576/82**
**10.08.83 CH 4356/83**

(43) Veröffentlichungstag der Anmeldung : **28.03.84 Patentblatt 84/13**

(45) Bekanntmachung des Hinweises auf die Patenterteilung : **17.07.85 Patentblatt 85/29**

(84) Benannte Vertragsstaaten : **AT BE CH DE FR GB IT LI NL**

(56) Entgegenhaltungen :
**EP-A- 0 045 571**
**CH-A- 512 442**
**DE-A- 2 205 791**
**DE-A- 2 752 096**
**DE-A- 2 851 024**
**DE-A- 2 952 719**
**DE-B- 1 280 835**

(73) Patentinhaber : **L. GIVAUDAN & CIE Société Anonyme**
**CH-1214 Vernier-Genève (CH)**

(72) Erfinder : **Gabbai, Albert, Dr.**
**5A rue de la Golette**
**CH-1217 Meyrin (CH)**
Erfinder : **De Polo, Karl-Fred, Dr.**
**35 avenue du Gros-Chêne**
**CH-1213 Onex (CH)**

(74) Vertreter : **Lederer, Franz, Dr. et al**
**Patentanwälte Dr. Lederer Franz Meyer-Roxlau Reiner F. Lucile-Grahn-Strasse 22**
**D-8000 München 80 (DE)**

**EP 0 103 896 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von p-tert.Butyl-α-methylhydrozimtaldehyd, also der Verbindung der Formel

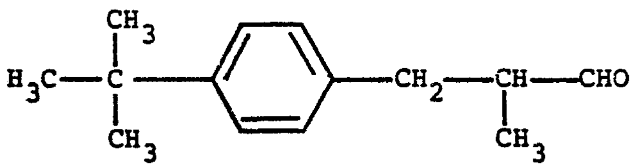

(I)

welche Verbindung einen bekannten Riechstoff darstellt.

Das Verfahren ist dadurch gekennzeichnet, dass man eine Verbindung der Formel

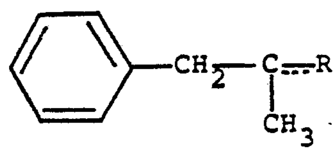

(II)

worin R einen als Enamin, Oxazolidin oder — vorzugsweise — Schiff'sche Base geschützten Formylrest bedeutet, und die gestrichelte Linie eine fakultative Bindung darstellt, t-butyliert und die Schutzgruppe hierauf abspaltet.

Die Verbindungen der Formel II sind neu und bilden einen weiteren Gegenstand der Erfindung. Sie sind in an sich bekannter Weise aus dem α-Methyl-hydrozimtaldehyd herstellbar.

So setzt man z. B. zwecks Bildung des Enamins den Aldehyd mit einem sekundären Amin in an sich bekannter Weise um. Geeignete sekundäre Amine sind Dialkylamine, z. B. Di-$C_{2-6}$-alkylamine, wie Diäthylamin, Dipropylamin, oder cyclische sekundäre Amine, wie z. B. Morpholin, Piperidin, Pyrrolidin. Bevorzugt sind Morpholin und Di-n-propylamin.

Man verwendet das Amin zweckmässigerweise in aequimolarer Menge, vorteilhaft aber in 10-20 %igem Ueberschuss. Die Enaminbildung wird durch Erhitzen, zweckmässigerweise auf Rückflustemperatur bewerkstelligt. Man arbeitet in Anwesenheit eines inerten Lösungsmittels, das mit dem bei der Reaktion gebildeten Wasser ein Azeotrop bildet, wie Cyclohexan, Benzol, Toluol oder Xylole, und gegebenenfalls in Gegenwart einer Säure, wie z. B. Toluolsulfonsäure, Camphersulfonsäure, Ameisensäure, Benzoesäure, Methansulfonsäure. Bevorzugt ist Ameisensäure. Man kann die Enaminbildung auch unter Zuhilfenahme eines Soxhlet-Extraktors durchführen, wobei das Azeotrop (Wasserschleppmittel-/$H_2O$) in diesem Soxhlet-Extraktor durch ein Trocknungsmittel, wie z. B. durch ein Molekularsieb, etc. perkoliert wird. Die Reaktionstemperatur richtet sich nach der Siedetemperatur des Lösungsmittel-Wasser-Azeotrops, sie liegt aber insbesondere im Bereich von 80°-140 °C.

Das Oxazolidinderivat II wird durch Umsetzung des Aldehyds mit Monoäthanolamin, Diäthanolamin oder N-Alkyl-N-äthanolaminen, zweckmässigerweise in äquimolaren Mengen, vorzugsweise in 10-30 %igem Ueberschuss der Aminkomponente erhalten. Man arbeitet im übrigen zweckmässigerweise wie oben angegeben.

Es hat sich gezeigt, dass die Enamine und Oxazolidine nach der azeotropen Entfernung des Reaktionswassers, in Toluol, Benzol oder Cyclohexan, etc. gelöst, mit kalter, gesättigter Kochsalzlösung gewaschen werden können. Nach dem Trocknen über Natriumsulfat und Einengen des Reaktionsgemisches können die rohen Enamine oder rohen Oxazolidine direkt für die Tertiärbutylierung eingesetzt werden.

Zwecks Bildung der Schiff'schen Base II setzt man den Aldehyd mit einem Primären Amin in an sich bekannter Weise um. Geeignete Amine sind : Alkylamine. z. B. $C_{1-6}$-Alkylamine, wie Methylamin, Aethylamin, Propylamin, alicyclische Amine wie z. B. Cyclohexylamin, etc., wobei Methylamin und 2-Butylamin bevorzugt sind.

Man arbeitet im übrigen wie oben angegeben.

Zwecks tert.Butylierung der Verbindung II wird die letztere Verbindung mit einer Verbindung umgesetzt, die das Carboniumion der Formel

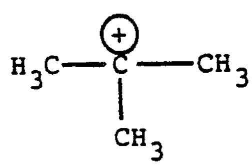

(III)

liefert.

Bevorzugte Verbindungen, welche das Carboniumion der allgemeinen Formel III liefern, sind Isobutylen, tert.Butylhalogenide, z. B. tert.-Butylchlorid, oder tert.Butylalkohol.

Die tert.Butylierung der Verbindung der allgemeinen Formel II erfolgt in Anwesenheit einer geeigneten Menge eines Friedel-Crafts Katalysators. Als Katalysatoren eignen sich die bekannten Friedel-Crafts Katalysatoren, wie z. B. Aluminiumchlorid, Eisenchlorid, Bortrifluorid, Schwefelsäure. Für die Zwecke der vorliegenden Erfindung ist Schwefelsäure besonders bevorzugt, wobei pro Mol der Verbindung II z. B. 2-10 Mole, bevorzugt 4-6 Mole konzentrierte Schwefelsäure verwendet werden.

Die Reaktion kann gegebenenfalls in Gegenwart eines inerten organischen Lösungsmittels durchgeführt werden. Als inerte organische Lösungsmittel eignen sich besonders Alkane, wie Hexan, Cyclohexan, chlorierte Kohlenwasserstoffe, wie Chloroform, Aethylendichlorid und Methylenchlorid, wobei Methylenchlorid bevorzugt ist. Die Temperatur der Alkylierungsreaktion liegt zweckmässigerweise zwischen etwa 0-20 °C, vorzugsweise zwischen etwa 0-10 °C.

Die Reaktionszeit kann in einem weiten Bereich variieren. Sie beträgt z. B. 1/2 Stunde bis mehrere Stunden.

Die Abspaltung der Schutzgruppe aus der p-tertiärbutylierten Verbindung II erfolgt zweckmässigerweise durch einfaches Verdünnen des Reaktionsgemisches mit Eis und Wasser. Die Anwesenheit eines mit Wasser nicht mischbaren, organischen Lösungsmittels wie Methylenchlorid, Aethylenchlorid, Hexan, Benzol, Toluol, Diisopropylaether, Diäthyläther u. a. ist zweckmässig.

Es sind zwar Verfahren zur Herstellung der Verbindung der Formel I bekanntgeworden, die ebenfalls eine t-Butylierungsstufe aufweisen, siehe z. B. DOS 2,952,719 vom 16. Juli 1981 und Europäische Patentpublikation Nr. 45 571 vom 10. Februar 1982. Verglichen mit den bekannten Verfahren verfügt das vorliegende Verfahren über folgende Vorteile :

Bei den bekannten Verfahren muss in einer letzten Stufe der Alkohol der Formel

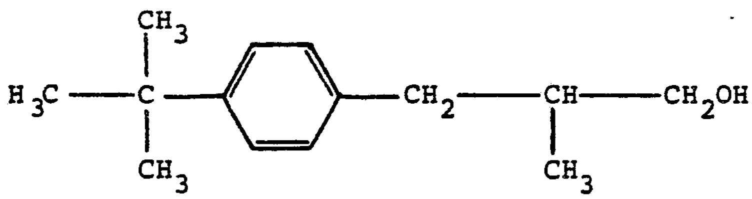

(IV)

wieder auf die Oxidationsstufe der Verbindung der Formel I gebracht werden. Diese Stufe weist folgende, ins Gewicht fallende Nachteile auf :

a) Sie ist energetisch unbefriedigend, da bei hohen Temperaturen gearbeitet werden muss.

b) Es tritt — im Falle hoher Konversionen — leicht Ueberoxidation ein ; damit ist Bildung der entsprechenden Säure verbunden. Darunter leidet die Ausbeute an I.

c) Andererseits kann aber auch — im Fale niedriger Konversionen — noch Ausgangsmaterial IV im Endprodukt I vorliegen ; dies erfordert eine schwierige (destillative) Trennung.

## Beispiel 1

In einen 4-Halsrundkolben, der mit Rührer, Thermometer, Tropftrichter und Wasserabscheider versehen ist, gibt man 74 g (0,5 Mol) α-Methylhydrozimtaldehyd und 150 ml Cyclohexan. Unter Rühren und Kühlen werden 30,54 g (0,5 Mol) Monoäthanolamin zugegeben. Man rührt eine halbe Stunde, gibt 0,3 g p-Toluolsulfonsäure zu und hält das Gemisch bei Rückflusstemperatur, bis die theoretische Menge Wasser abgeschieden ist. Nun gibt man 0,5 g Kaliumcarbonat zu und rührt 10 Minuten. Hierauf filtriert man und entfernt das Lösungsmittel am Rotationsverdampfer. Man erhält 94,4 g des Oxazolidins (theoretische Ausbeute 95,63 g).

Das Produkt wird über einer 6 cm Vigreux-Kolonne rektifiziert. Die Ausbeute an reinem Produkt beträgt 56,5 g (59,1 % der Theorie). Sdp., 98 °C bei 0,3 Torr ; $n_D^{20}$ = 1,527 0. Da sich bei der destillativen Reinigung das Oxazolidin teilweise zersetzt, wird für die nächste Stufe vorzugsweise das rohe Oxazolidin eingesetzt. Aus dem IR- und NMR-Spektrum ist ersichtlich, dass das Reaktionsprodukt ein Gleichgewichtsgemisch (1 : 1) von geschlossener Oxazolidinform und offener Form (Schiff'sche Base) darstellt :

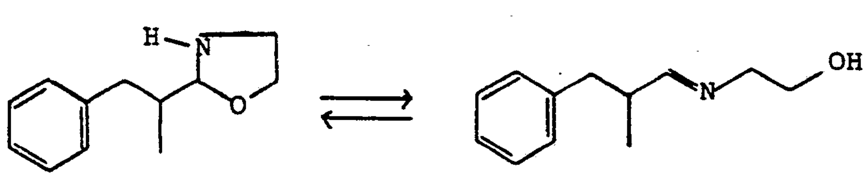

Die Tertiärbutylierung wird in einem 500 ml 4-Halssulfierkolben durchgeführt, der mit Thermometer, Flügelrührer, Gaseinleitungrohr und einem, in gesättigte Kochsalzlösung eintauchenden Steigrohr versehen ist. Die Einleitung des Isobutylens erfolgt aus einer Gasdruckdose, die auf einer Waage steht.

In das Reaktionsgefäss gibt man 62 g (0,6 Mol) konzentrierte Schwefelsäure. Unter Kühlung mit einer Trockeneis-Isopropanol-Kältemischung lässt man während 30 Minuten zwischen 0 °C und 5 °C die

Lösung von 19,2 g Oxazolidin in 80 ml Methylenchlorid zutropfen. Man leitet nun während einer Stunde bei einer Temperatur von 18-20 °C 7,4 g Isobutylen ein. Anschliessend wird das Reaktionsgemisch noch 30 Minuten bei 20° gerührt. Man giesst hierauf aus 550 g Eis. Es bildet sich dabei vorerst ein pastenförmiger Niederschlag, der nach 2 Stunden Stehenlassen in Lösung gegangen ist. Das Reaktionsgemisch wird in einen Scheidetrichter übergeführt und die wässrige Phase noch zweimal mit Methylenchlorid extrahiert. Die organische Phase wird neutral gewaschen, über Natriumsulfat getrocknet und auf dem Rotationsverdampfer eingedampft. Man erhält 23,9 g des rohen Aldehyds I (theoretische Ausbeute 20,5 g). Die fraktionierte Destillation ergibt 10,3 g reines I, Sdp. 100-102 °C/l Torr ; Ausbeute 50,2 % der Theorie.

## Beispiel 2

In einen 500 ml 4-Halsrundkolben, der mit Rührer, Wasserabscheider, Tropftrichter und Thermometer versehen ist, gibt man 74 g (0,5 Mol) α-Methylhydrozimtaldehyd und 150 ml Cyclohexan. Man gibt unter starkem Rühren aus dem Tropftrichter 46 g (0,65 Mol) Pyrrolidin, in dem 0,1 g p-Toluolsulfonsäure aufgelöst sind, langsam zu. Die Temperatur des Reaktionsgemisches steigt hierauf auf 35 °C an. Nach der Zugabe, die 30 Minuten erfordert, wird noch 30 Minuten bei 35° weitergerührt. Anschliessend wird ein Wasserabscheider aufgesetzt und das Reaktionswasser bei Rückflusstemperatur ausgekreist. nach 2 1/2 Stunden haben sich auf diese Weise 9,5 ml Wasser abgeschieden. Man setzt dem Reaktionsgemisch noch eine geringe Menge Kaliumcarbonat zu, rührt und filtriert. Man erhält auf diese Weise 104 g rohres Enamin. Die fraktionierte Destillation ergibt 53 g Enamin. Sdp. 124-128 °C/7 Torr ; $n_D^{20}$ = 1,543 4, Ausbeute 52,7 %.

Die tert.Butylierung wird in einem 250 ml 4-Halsrundkolben, der mit Rührer, Thermometer, Gaseinleitungsrohr und Steigrohr versehen ist, durchgeführt.

In das Reaktionsgefäss gibt man 55 g konzentrierte Schwefelsäure. Unter Kühlen mittels Eis-Kochsalz-Kältemischung wird bei einer Temperatur von 5 °C-10 °C während 3/4 Stunden die Lösung von 20,5 g des oben hergestellten Enamins in 80 ml Methylenchlorid zugetropft. Das Reaktionsgemisch färbt sich nun tiefbraun. Nun wird der Tropftrichter durch das Gaseinleitungsrohr ersetzt und es werden während einer Stunde bei 19-20 °C 8,3 g Isobutylen eingeleitet. Nach Beendigung der Einleitung wird das Reaktionsgemisch noch 1 1/2 Stunden bei Zimmertemperatur gerührt. Man giesst hierauf unter Rühren zu 500 ml entmineralisiertem Wasser und rührt das Gemisch während 30 Min. Die organische Phase wird im Scheidetrichter abgetrennt und die wässrige Phase nochmals mit Methylenchlorid extrahiert. Die organischen Phasen werden neutral gewaschen, getrocknet und am Rotationsverdampfer eingedampft. Man erhält 18,7 g rohes I. Die Destillation im Kugelrohr ergibt 8,6 g reines I vom Sdp. 100-102 °C/l Torr ; die Ausbeute beträgt 42 %.

## Beispiel 3

In einen 250 ml 4-Halsrundkolben, der mit Rührer, Thermometer und Gaseinleitungsrohr versehen ist, gibt man 44,4 g (0,3 Mol) α-Methylhydrozimtaldehyd, 60,7 g (0,6 Mol) Dipropylamin und 0,3 g Toluolsulfonsäure und 100 ml Cyclohexan. Man gibt einige Siedesteine zu und erwärmt das Reaktionsgemisch 2 1/2 Stunden am Wasserabscheider auf Rückflusstemperatur. Die Reaktion wird gaschromatographisch verfolgt (Kolonne 2 % OV 101 (Methylsilicon der Applied Science Laboratories, Inc., P.O. Box 440, State College P.A.) auf Chromosorb G, DMCS AW 80-100 mesh. Temperatur 200 °C, Gas ; Helium ; Detektor FID 250 °C). Es werden 4,2 ml Wasser abgeschieden (theoretische Menge 5,4 ml). Das Reaktionsgemisch wird bei 50 °C und 30 Torr auf dem Rotationverdampfer eingedampft. Man erhält auf diese Weise 73,4 g rohes Enamin (theoretische Menge 69,4 g), welche direkt der tert.Butylierung unterworfen werden.

Eine kleine Menge des erhaltenen Dipropyl-Enamins wird durch fraktionierte Destillation gereinigt. Das reine Produkt weist einen Siedepunkt von 100 °C bei 0,5 Torr auf ; $n_D^{20}$ = 1,503 0.

Die Tertiärbutylierung wird in einem 350 ml 4-Halssulfierkolben durchgeführt, der mit Flügelrührer, Gaseinleitungsrohr und einem in gesättigte Kochsalzlösung eintauchenden Ueberdrucksteigrohr versehen ist. Das Isobutylen wird aus einer, auf einer Waage stehenden Gasdruckdose eingeleitet.

In das Reaktionsgefäss wägt man 200 g konzentrierte Schwefelsäure ein. Dazu tropft man langsam bei − 2 °C bis 0 °C unter Kühlen mit einem Eis-Kochsalz-Kältegemisch eine Lösung von 73,4 g des oben hergestellten rohen Dipropyl-Enamins in 80 ml Methylenchlorid, innerhalb von 2 1/2 Stunden zu. Das Reaktionsgemisch färbt sich rotbraun. Nun wird der Tropftrichter durch das Gaseinleitungsrohr ersetzt und innert 1 3/4 Stunden werden zwischen 17 und 19 °C 22,4 g Isobutylen eingeleitet. Nach Beendigung des Einleitens wird das Reaktionsgemisch noch eine Stunde bei Zimmertemperatur gerührt. Hierauf gibt man es unter Rühren auf 240 g Eis. Die organische Phase wird abgetrennt und die wässrige Phase noch dreimal mit Methylenchlorid extrahiert. Die vereinigten organischen Phasen werden mit ca. 20 ml 50 %iger Kalilauge neutralisiert. Man wäscht neutral und trocknet über Natriumsulfat. Man dampft am Rotationsverdampfer ein und erhält auf diese Weise 63,1 g rohes I. Durch fraktionierte Destillation des Rohproduktes erhält man 27,8 g reines I (Ausbeute 45,4 %).

Beispiel 4

40,2 g 2-Butylamin werden langsam zu einer gerührten Lösung von 75,7 g Methylphenylpropanal in 125 ml Diisopropyläther gegeben. Das bei der Reaktion gebildete Wasser wird mittels eines Wasserabscheiders abgetrennt. Nach 5 Stunden Reaktionszeit wird das Lösungsmittel abgedampft und das rohe Imin (105 g) kurzwegdestilliert, wobei 86 g N-(2-Methyl-3-phenylpropyliden)-2-butylamin in einer Reinheit von 96,4 % anfallen ; die Ausbeute beträgt 91,2 %.

21,3 g des Imins werden innert 1 1/2 Stunden unter Rühren zu 62 g 95 % $H_2SO_4$ gegeben. man kühlt das Gemisch während der Zugabe auf 4 °C ab. In die gerührte Lösung werden hierauf innert 1 1/2 Stunden bei 5 °C 2,7 l Isobutylen eingeleitet ; hierauf gibt man 100 ml Methylenchlorid zu. Man gibt das Reaktionsgemisch unter Rühren auf ein Gemisch von 100 ml methylenchlorid und 522 g 10 %iger Natronlauge, wobei die Temperatur unterhalb von 15 °C gehalten wird. Die organische Phase wird abgetrennt und mit Wasser bis zu schwach alkalischer Reaktion gewaschen. Nach dem Trocknen und Eindampfen des Lösungsmittels erhält man 29,1 g rohes, paratertiobutyliertes Imin. Eine Destillation über eine 15 cm-Widmerkolonne liefert 12,5 g N-[p-tert.Butyl-(2-methyl-3-phenylpropyliden)]2-butylamin in einer Reinheit von 91 % ; Siedepunkt 106 °C/0,6 Torr. 4,3 g Ausgangsmaterial werden zurückgewonnen. Durch Zugabe von 60 ml 2N $H_2SO_4$ bei 5 °C erhält man aus dem Imin das Produkt I in einer Ausbeute von 95 %.

21 g des Imins werden innert einer Stunde in 62 g 95 %ige $H_2SO_4$ gegeben, wobei dauernd gerührt wird und die Temperatur unterhalb 8 °C gehalten wird. Nun werden bei 5 °C unter Rühren 3,86 l Isobutylen eingeleitet, welche Zugabe innert 2 1/4 Stunden erfolgt. Hierauf gibt man bei 25 °C 40 ml Methylenchlorid und 40 ml Wasser zu. Nach Aufarbeitung, Abdampfen des Lösungsmittels und Kurzwegdestillation erhält man das 2-(p-tert.-Butylbenzyl)propanal in einer Ausbeute von 85 %.

Die Ausbeuten an I ausgehend von verwandten Schiff'schen Basen sind :

| | |
|---|---|
| N-(2-Methyl-3-phenylpropyliden)methylamin | 82 % |
| N-(2-Methyl-3-phenylpropyliden)cyclohexylamin | 75 % |
| N-(2-Methyl-3-phenylpropyliden)anilin | 95 % |
| N-(2-Methyl-3-phenylpropyliden)-1-butylamin | 70,7 % |
| N-(2-Methyl-3-phenylpropyliden)-tert.butylamin | 64,5 % |

Beispiel 5

In einen 250 ml Schliff-Rundkolben gibt man 74,1 g α-Methylhydrozimtaldehyd, 80 ml Cyclohexan, und, unter Umschwenken, 65,4 g Morpholin. Das Gemisch erwärmt sich auf 57 °C. Der Wasserabscheider wird aufgesetzt und das Reaktionswasser wird bei Rückflusstemperatur ausgekreist. Nach drei Stunden Reaktionsdauer haben sich auf diese Weise 9,2 ml Wasser abgeschieden. Eine gaschromatographische Analyse zeigt, dass noch rund 5 % unveränderter Ausgangsaldehyd vorhanden sind. Durch Perkolation des siedenden Wasserschleppmittels im Soxhlet-Extraktor über 5 g 3A-Molekularsieb wird auch das restliche Ausgangsprodukt vollständig umgesetzt.

Nach Eindampfen des Wasserschleppmittels am Rotationsverdampfer erhält man 117,8 g rohes Enamin. Dieses wird im Kugelrohr einer Kurzwegdestillation unterworfen, wobei man 101,7 g reines Enamin erhält : Sdp. 125 °C bei 0,06 Torr ; $n_D^{20} = 1{,}536\,2$ ; Ausbeute 92,8 %.

In einen 500 ml Sulfierkolben, der mit Rührer, Thermometer und einem Tropftrichter versehen ist, werden 155 g konzentrierte Schwefelsäure eingewogen. Unter Rühren und Kühlen mit einem Eis-Kochsalz-Kältegemisch werden unterhalb 5 °C 54,3 g Morpholin-Enamin eingetropft. Anschliessend wird der Tropftrichter durch ein Gaseinleitungsrohr, das in das Reaktionsgemisch eintaucht, ersetzt. Der Sulfierkolben wird zudem mit einem in gesättigte Kochsalzlösung eintauchenden Steigrohr verbunden. Aus einer auf einer Waage stehenden Gasdruckdose werden anschliessend unter Rühren unterhalb 10 °C während 80 Minuten 23,8 g Isobutylen eingeleitet. Man rührt während einer Stunde bei 10 °C und giesst anschliessend auf ein Gemisch von 100 ml Aether und 1 kg Eis.

Man trennt die organische Phase ab und extrahiert die wässrige Phase zweimal mit Aether. Die vereinigten organischen Phasen werden abgetrennt und neutralgewaschen.

Nach dem Trocknen über Natriumsulfat und Eindampfen des Lösungsmittels erhält man 47,5 g Rohprodukt. Dieses wird im Kugelrohr kurzwegdestilliert. Man erhält 40,6 g Aldehydgemisch. Die gaschromatographische Analyse ergibt, dass das Gemisch aus 60,3 % I und 37 % α-Methylhydrozimtaldehyd besteht. Ausbeute : 47,8 % an I. Die weitere Reinigung geschieht durch Kolonnendestillation.

**Patentansprüche** (für die Vertragsstaaten : BE, CH, DE, FR, GB, IT, LI, NL)

1. Verfahren zur Herstellung von p-tert.Butyl-α-methylhydrozimtaldehyd, dadurch gekennzeichnet, dass man eine Verbindung der Formel

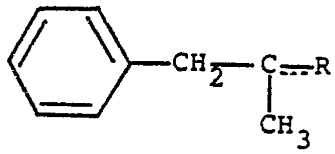

(II)

worin R einen als Enamin, Oxazolidin oder Schiff'sche Base geschützten Formylrest bedeutet, und die gestrichelte Linie eine fakultative Bindung darstellt, t-butyliert und die Schutzgruppe hierauf entfernt.

2. Verfahren nach Anspruch 1, dadurch gekennzeichnet, dass man unter Zuhilfenahme von Isobutylen, eines tert. Butylhalogenids oder von tert. Butylalkohol t-butyliert.

3. Verfahren nach Anspruch 2, dadurch gekennzeichnet, dass man die Reaktion in Gegenwart von konzentrierter $H_2SO_4$ durchführt.

4. Verfahren nach einem der Ansprüche 1-3, dadurch gekennzeichnet, dass man die Verbindung der Formel II durch Umsetzung von α-Methylhydrozimtaldehyd mit einem primären oder sekundären Amin, mit Monoäthanolamin, Diäthanolamin oder mit einem N-Alkyl-N-aethanolamin herstellt.

5. Verbindungen der Formel

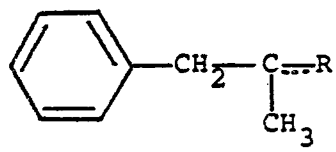

(II)

worin R einen als Enamin, Oxazolidin oder Schiff'sche Base geschützten Formylrest bedeutet, und die gestrichelte Linie eine fakultative Bindung darstellt.

6. Verbindungen des Formel II, worin R einen als Enamin geschützten Formylrest bedeutet.
7. Verbindungen der Formel II, worin R einen als Oxazolidin geschützten Formylrest bedeutet.
8. Verbindungen der Formel II, worin R einen als Schiff'sche Base geschützten Formylrest bedeutet.
9. Enamin aus α-Methylhydrozimtaldehyd und Pyrrolidin.
10. Enamin aus α-Methylhydrozimtaldehyd und Dipropylamin.
11. Enamin aus α-Methylhydrozimtaldehyd und Morpholin.
12. Oxazolidin aus α-Methylhydrozimtaldehyd und Monoäthanolamin.
13. Schiff'sche Base aus α-Methylhydrozimtaldehyd und 2-Butylamin.
14. Schiff'sche Base aus α-Methylhydrozimtaldehyd und Methylamin.
15. Schiff'sche Base aus α-Methylhydrozimtaldehyd und Cyclohexylamin.
16. Schiff'sche Base aus α-Methylhydrozimtaldehyd und Anilin.
17. Schiff'sche Base aus α-Methylhydrozimtaldehyd und n-Butylamin.
18. Schiff'sche Base aus α-Methylhydrozimtaldehyd und tert. Butylamin.

**Patentansprüche** (für den Vertragsstaat AT)

1. Verfahren zur Herstellung von p-tert.Butyl-α-methylhydrozimtaldehyd, dadurch gekennzeichnet, dass man eine Verbindung der Formel

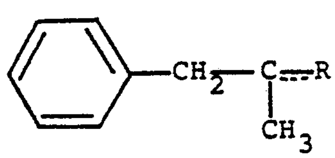

(II)

worin R einen als Enamin, Oxazolidin oder Schiff'sche Base geschützten Formylrest bedeutet, und die gestrichelte Linie eine fakultative Bindung darstellt, t-butyliert und die Schutzgruppe hierauf entfernt.

2. Verfahren nach Anspruch 1, dadurch gekennzeichnet, dass man unter Zuhilfenahme von Isobutylen, eines tert. Butylhalogenids oder von tert. Butylalkohol t-butyliert.

3. Verfahren nach Anspruch 2, dadurch gekennzeichnet, dass man die Reaktion in Gegenwart von konzentrierter $H_2SO_4$ durchführt.

4. Verfahren nach einem der Ansprüche 1-3, dadurch gekennzeichnet, dass man die Verbindung der Formel II durch Umsetzung von α-Methylhydrozimtaldehyd mit einem primären oder sekundären Amin, mit Monoäthanolamin, Diäthanolamin oder mit einem N-Alkyl-N-aethanolamin herstellt.

**Claims** (for the Contracting States : BE, CH, DE, FR, GB, IT, LI, NL)

1. A process for the manufacture of p-tert.butyl-α-methylhydrocinnamaldehyde, characterized by butylating a compound of the formula

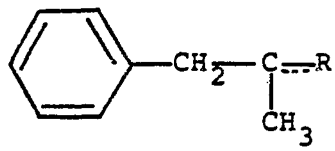

(II)

wherein R signifies a formyl residue protected as an enamine, oxazolidine or Schiff's base, and the dotted line represents an optional bond,
and thereupon removing the protecting group.

2. A process according to claim 1, characterized in that the t-butylation is carried out with the aid of isobutylene, a tert. butyl halide or of tert. butyl alcohol.

3. A process according to claim 2, characterized in that the reaction is carried out in the presence of concentrated $H_2SO_4$.

4. A process according to any one of claims 1-3, characterized in that the compound of formula II is prepared by reacting α-methylhydrocinnamaldehyde with a primary or secondary amine, with monoethanolamine, diethanolamine or with a N-alkyl-N-ethanolamine.

5. Compounds of the formula

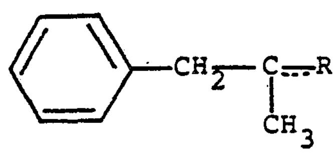

(II)

wherein R signifies a formyl residue protected as an enamine, oxazolidine or Schiff's base, and the dotted line represents an optional bond.

6. Compounds of formula II, wherein R signifies a formyl residue protected as an enamine.

7. Compounds of formula II, wherein R signifies a formyl residue protected as an oxazolidine.

8. Compounds of formula II, wherein R signifies a formyl residue protected as a Schiff's base.

9. The enamine from α-methylhydrocinnamaldehyde and pyrrolidine.

10. The enamine from α-methylhydrocinnamaldehyde and dipropylamine.

11. The enamine from α-methylhydrocinnamaldehyde and morpholine.

12. The oxazolidine from α-methylhydrocinnamaldehyde and monoethanolamine.

13. The Schiff's base from α-methylhydrocinnamaldehyde and 2-butylamine.

14. The Schiff's base from α-methylhydrocinnamaldehyde and methylamine.

15. The Schiff's base from α-methylhydrocinnamaldehyde and cyclohexylamine.

16. The Schiff's base from α-methylhydrocinnamaldehyde and aniline.

17. The Schiff's base from α-methylhydrocinnamaldehyde and n-butylamine.

18. The Schiff's base from α-methylhydrocinnamaldehyde and tert. butylamine.

**Claims** (for the Contracting State AT)

1. A process for the manufacture of p-tert.butyl-α-methylhydrocinnamaldehyde, characterized by t-butylating a compound of the formula

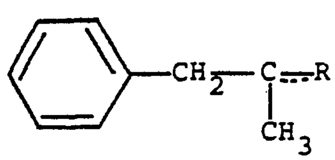

(II)

wherein R signifies a formyl residue protected as an enamine, oxazolidine or Schiff's base, and the dotted line represents an optional bond,
and thereupon removing the protecting group.

2. A process according to claim 1, characterized in that the t-butylation is carried out with the aid of isobutylene, a tert. butyl halide or of tert. butyl alcohol.

3. A process according to claim 2, characterized in that the reaction is carried out in the presence of concentrates $H_2SO_4$.

4. A process according to any one of claims 1-3, characterized in that the compound of formula II is prepared by reacting α-methylhydrocinnamaldehyde with a primary or secondary amine, with monoethanolamine, diethanolamine or with a N-alkyl-N-ethanolamine.

**Revendications** (pour les Etats contractants : BE, CH, DE, FR, GB, IT, LI, NL)

1. Procédé de préparation de l'aldéhyde p-tert-butyl-α-méthyl-hydrocinnamique, caractérisé en ce que l'on soumet un composé de formule

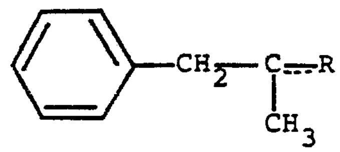

(II)

dans laquelle R représente un groupe formyle protégé à l'état d'énamine, d'oxazolidine ou de base de Schiff, et le trait interrompu représente une liaison facultative,
à tert-butylation, après quoi on élimine le groupe protecteur.

2. Procédé selon la revendication 1, caractérisé en ce que la tert-butylation est réalisée à l'aide d'isobutylène, d'un halogénure de tert-butyle ou de l'alcool tert-butylique.

3. Procédé selon la revendication 2, caractérisé en ce que l'on effectue la réaction en présence de $H_2SO_4$ concentré.

4. Procédé selon l'une des revendications 1 à 3, caractérisé en ce que l'on prépare le composé de formule II par réaction de l'aldéhyde α-méthyl-hydrocinnamique avec une amine primaire ou secondaire, avec la monoéthanolamine, la diéthanolamine ou une N-alkyl-N-éthanolamine.

5. Composés de formule

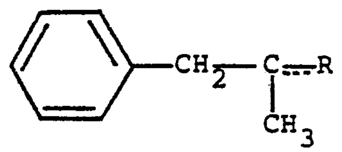

(II)

dans laquelle R représente un groupe formyle protégé à l'état d'énamine, d'oxazolidine ou de base de Schiff, et le trait interrompu représente une liaison facultative.

6. Composés de formule II dans laquelle R représente un groupe formyle protégé à l'état d'énamine.

7. Composés de formule II dans laquelle R représente un groupe formyle protégé à l'état d'oxazolidine.

8. Composés de formule II dans laquelle R représente un groupe formyle protégé à l'état de base de Schiff.

9. L'énamine de l'aldéhyde α-méthyl-hydrocinnamique et de la pyrrolidine.

10. L'énamine de l'aldéhyde α-méthyl-hydrocinnamique et de la dipropylamine.

11. L'énamine de l'aldéhyde α-méthyl-hydrocinnamique et de la morpholine.

12. L'oxazolidine de l'aldéhyde α-méthyl-hydrocinnamique et de la monoéthanolamine.

13. La base de Schiff de l'aldéhyde α-méthyl-hydrocinnamique et de la 2-butylamine.

14. La base de Schiff de l'aldéhyde α-méthyl-hydrocinnamique et de la méthylamine.

15. La base de Schiff de l'aldéhyde α-méthyl-hydrocinnamique et de la cyclohexylamine.

16. La base de Schiff de l'aldéhyde α-méthyl-hydrocinnamique et de l'aniline.

17. La base de Schiff de l'aldéhyde α-méthyl-hydrocinnamique et de la n-butylamine.

18. La base de Schiff de l'aldéhyde α-méthyl-hydrocinnamique et de la tert.-butylamine.

**Revendications** (pour l'Etat contractant AT)

1. Procédé de préparation de l'aldéhyde p-tert-butyl-α-méthyl-hydrocinnamique, caractérisé en ce que l'on soumet un composé de formule

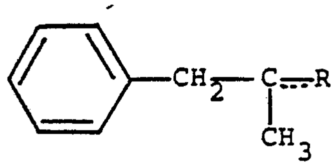

(II)

dans laquelle R représente un groupe formyle protégé à l'état d'énamine, d'oxazolidine ou de base de Schiff, et le trait interrompu représente une liaison facultative,
à tert-butylation, après quoi on élimine le groupe protecteur.

2. Procédé selon la revendication 1, caractérisé en ce que la tert-butylation est réalisée à l'aide d'isobutylène, d'un halogénure de tert-butyle ou de l'alcool tert-butylique.

3. Procédé selon la revendication 2, caractérisé en ce que l'on effectue la réaction en présence de $H_2SO_4$ concentré.

4. Procédé selon l'une des revendications 1 à 3, caractérisé en ce que l'on prépare le composé de formule II par réaction de l'aldéhyde α-méthyl-hydrocinnamique avec une amine primaire ou secondaire, avec la monoéthanolamine, la diéthanolamine ou une N-alkyl-N-éthanolamine.